# EUROPÄISCHE PATENTANMELDUNG

(11) **EP 1 900 799 A2**
(43) Veröffentlichungstag der Anmeldung: **19.03.2008**
(21) Anmeldenummer: 07017061.8
(22) Anmeldetag: 31.08.2007
(51) Int. Cl.: C11B 3/10, C11B 3/08, C11D 3/39, C07C 41/36, C07C 41/38

(54) **Verfahren zur Behandlung von Fettstoffen mit Kieselgel und Wasserstoffperoxid**

(30) Priorität: 08.09.2006 EP 06018838
(71) Anmelder: Cognis IP Management GmbH, 40589 Düsseldorf (DE)
(72) Erfinder: Richard-Elsner, Christiane, 40595 Düsseldorf (DE)

(57) **Zusammenfassung**

Die vorliegende Erfindung betrifft ein Verfahren zum Behandeln von Fettstoffen mit Kieselgel und Wasserstoffperoxid, wobei dieses Verfahren den Vorteil hat, dass sowohl unerwünscht hohe pH-Werte der Fettstoffe erniedrigt werden können, als auch dass eine Bleichwirkung erreicht werden kann.

## Beschreibung

Die vorliegende Erfindung betrifft ein Verfahren zum Behandeln von Fettstoffen mit Kieselgel und Wasserstoffperoxid, wobei dieses Verfahren den Vorteil hat, dass sowohl unerwünscht hohe pH-Werte der Fettstoffe erniedrigt werden können, als auch dass eine Bleichwirkung erreicht werden kann.

Organische Substanzen, besonders Inhaltsstoffe für Kosmetika, Reiniger oder Pharmaprodukte, sollen für die Anwendung möglichst farblos sein, damit sie beliebig in die jeweiligen Rezepturen eingearbeitet werden können ohne zu einer Grundfärbung beizutragen. Aus diesem Grunde werden organische Substanzen, besonders für die o. a. Einsatzgebiete, einem so genannten Bleichprozess unterworfen. Im Rahmen dieses Bleichprozesses wird die Farbe der Substanz aufgehellt, vorzugsweise wird die Substanz im sichtbaren Licht farblos. Als Bleichmittel werden z. B. Bleicherden verwendet, wobei es sich um natürliche Magnesiumaluminiumsilikate handelt. In anderen Fällen werden Aktivkohlen zur Entfärbung eingesetzt. Weitere Entfärbungsverfahren sind beispielsweise Adsorption an polymeren Absorberharzen oder die Destillation. In vielen Fällen wird auch eine Peroxoverbindung und vorzugsweise Wasserstoffperoxid in wässriger Lösung zur Entfärbung eingesetzt. Der Entfärbungsgrad ist bei dieser Anwendung aber häufig nicht ausreichend. Außerdem ist Wasserstoffperoxid sicherheitstechnisch kritisch, da es leicht Sauerstoff abspaltet. Es ist allerdings aus der Literatur bekannt, beispielsweise aus der DE 41 42 592, dass man oberflächenaktive Verbindungen mit Wasserstoffperoxid bleichen kann und dies in Gegenwart von so genannten Bleichboostem tut, nämlich Erdalkaliionen, Zinkionen und/oder Alkalisilikaten. Die DE 41 42 592 nennt eine Vielzahl von oberflächenaktiven Verbindungen, die mittels Persauerstoff in Gegenwart der bekannten Bleichbooster gebleicht werden können, wobei allerdings als geeignete Silikate ausschließlich Alkalisilikate in Frage kommen. Des Weiteren nennt die DE 41 42 592 auch die Mitverwendung von Erdalkaliionen und insbesondere Magnesiumionen als geeignete Bleichbooster zur Bleiche von oberflächenaktiven Verbindungen.

Die im vorigen Absatz genannten organischen Substanzen enthalten in vielen Fällen herstellungsbedingt alkalische Verunreinigungen, haben also einen unerwünscht hohen pH-Wert. Für die weitere Verwendung ist es in der Regel erforderlich, diesen unerwünscht hohen pH-Wert zu erniedrigen. Dies erfordert in vielen industriellen Prozessen die Zugabe von Säuren, um einen unerwünscht hohen pH-Wert eines Rohproduktes abzusenken.

Die zwei in den vorigen Absätzen genannten Probleme (unerwünscht hoher pH-Wert und die Notwendigkeit eine Bleichung durchzuführen) treten insbesondere bei Fettstoffen auf. Der Begriff Fettstoffe wird in der vorliegenden Schrift noch genauer definiert. Insbesondere treten diese beiden Probleme bei Fettstoffen auf, die herstellungsbedingt als Rohprodukt einen unerwünscht hohen pH-Wert aufweisen. Dies ist insbesondere bei solchen Fettstoffen der Fall, bei deren Herstellung Basen, wie z. B. Hydroxide, wie z. B. Alkalihydroxide, wie z. B. NaOH oder KOH, eingesetzt werden. Diese Basen werden z. B. als Katalysatoren eingesetzt. Z. B. werden bei der Herstellung von Fettstoffen, die Ethoxylreste oder Propoxylreste oder Oligoethyoxyketten oder Oligopropoxyketten enthalten, oft Katalysatoren, z. B. die genannten Hydroxide, eingesetzt. Diese werden z. B. eingesetzt, um den Ausgangsstoff Ethylenoxid oder Propylenoxid zu den genannten Ethoxylresten oder Propoxylresten oder Oligoethyoxyketten oder Oligopropoxyketten umzuwandeln.

Ein Verfahren zum Bleichen von einem pflanzlichen Öl, dadurch gekennzeichnet, dass man das Öl mit Kieselgel in Kontakt bringt, bis eine Entfärbung eingetreten ist, ist bekannt **(**Gopala Krishna AG: "A method for bleaching rice bran oil with silica gel", Journal of the american oil chemists' society, AOCS Press, Champaign, IL, US, Bd. 69, Nr. 12, 1. Dezember 1992, Seiten 1257-1259**)**

Die Verwendung von H₂O₂ als Bleichmittel für ein Öl oder einen Fettstoff ist im Stand der Technik bekannt **(**Hodgson AS.: "Bailey's industrial oil and fat products - Vol. 4: Edible oil and fat products: Processing Technology" 1996, Bailey's industrial oil and fat products, New York, Wiley & Sons, US **und** WO 01/83655 A (Prolab Technologies Inc. (CA), Jollez Paul (CA), Garro Juan Miguel (CA) 8. November 2001)

Der vorliegenden Erfindung liegt die Aufgabe zu Grunde, ein Verfahren bereit zu stellen, das es erlaubt, bei Fettstoffen sowohl unerwünscht hohe pH-Werte zu erniedrigen als auch eine Bleichwirkung zu erzielen.

Dabei soll vorzugsweise mit H₂O₂ gebleicht werden, wobei die Bleichwirkung des H₂O₂ verstärkt werden soll.

Es wurde gefunden, dass die Verwendung von Kieselgelen die oben skizzierte Aufgabe zu lösen vermag.

Ein Gegenstand der vorliegenden technischen Lehre ist daher ein Verfahren zur Behandlung von Fettstoffen, die mindestens 6 C-Atome im Molekül aufweisen, dadurch gekennzeichnet, dass man die Fettstoffe mit einem H₂O₂ enthaltenden Bleichmittel sowie mit Kieselgel in Kontakt bringt, wobei die Fettstoffe bevorzugt so lange mit dem H₂O₂ enthaltenden Bleichmittel sowie mit dem Kieselgel in Kontakt bleiben, bis eine Entfärbung eingetreten ist, und wobei bevorzugt anschließend das Kieselgel entfernt und wobei die Fettstoffe, wenn sie in fester Form vorliegen, bevorzugt vor dem in Kontakt Bringen dem H₂O₂ enthaltenden Bleichmittel sowie mit dem Kieselgel unter Rühren auf Temperaturen oberhalb des Schmelzpunktes der Fettstoffe erwärmt werden.

In einer besonders bevorzugten Ausführungsform wird in einem ersten Schritt die erwärmte oberflächenaktive Verbindung mit Kieselgel behandelt und anschließend diese Mischung mit dem H₂O₂ enthaltenen Bleichmittel gebleicht. Am Ende des Bleichprozesses wird das Kieselgel beispielsweise durch Filtrieren oder jedes andere, dem Fachmann bekannte alternative Verfahren entfernt.

Eine weitere ebenfalls geeignete Variante des Verfahrens besteht darin, die oberflächenaktive Verbindung zunächst zu erwärmen, wobei neben dem H₂O₂ gleichzeitig das Kieselgel enthalten ist. In beiden Fällen werden hervorragend gebleichte Substrate erhalten.
Als Bleichmittel werden vorzugsweise wässerige, alkalisch eingestellte H₂O₂-Lösungen verwendet. Typischerweise enthalten diese bis zu 30 Gew.-% H₂O₂.

Das Verfahren eignet sich sowohl für die Bleiche eines Fettstoffes, als auch der Bleiche eine Mischung von Fettstoffen. Allerdings muss im ersten Schritt immer eine Erwärmung auf eine Temperatur oberhalb des Schmelzpunktes des Fettstoffes bzw. der Fettstoffmischung erfolgen. Für den Fall, dass nur bei Raumtemperatur (21°C) flüssige Fettstoffe vorliegen erübrigt sich natürlich eine Erwärmung. Vorzugsweise werden Fettstoffe bei dem erfindungsgemäßen Verfahren auf 50 bis 100°C erwärmt.

Ein weiterer Gegenstand der vorliegenden Erfindung ist die Verwendung einer Kombination aus einem H₂O₂ enthaltenden Bleichmittel und Kieselgel zur Erniedrigung eines unerwünscht hohen pH-Wertes und zur Bleichung von Fettstoffen, bevorzugt in dem erfindungsgemäßen Verfahren oder in einem seiner bevorzugten oder besonderen Ausführungsformen.

Unter Kieselgelen oder Silicagelen versteht man kolloidale geformte oder ungeformte Kieselsäure von elastischer bis fester Konsistenz mit lockerer bis dichter Porenstruktur und hohem Adsorptionsvermögen für Gase, Dämpfe u. Flüssigkeiten. In den Kieselgelen liegt die Kieselsäure in Form hochkondensierter Polykieselsäuren mit oberflächenreicher Blattstruktur vor (Kiesel-Xerogele); auf der Oberfläche der Kieselgelteilchen finden sich Siloxan und/oder Silanol-Gruppen. Die Herstellung der Kieselgele erfolgt aus Wasserglas durch Umsetzung mit Mineralsäuren. Bei den so gebildeten und ggf. als Kieselsole technisch eingesetzten Kieselsäure-Hydrosolen kann bei entsprechendem Temperatur- und pH-Wert die Umhüllung der kolloiddispersen Kieselsäure-Teilchen mit Wasser so weit gehen, dass das inkohärente System zu einem Gel erstarrt, bei dem die dispersen Kieselsäuren netz- od. wabenartig (oberflächenreiche Blattstruktur) im Wasser angeordnet sind. Die bei der Umsetzung gleichfalls entstandenen Salze (z.B. Na₂SO₄) müssen ausgewaschen werden. Je nach pH-Wert, bei dem das Waschen erfolgt, erhält man bei saurer (neutraler, basischer) Reaktion engporige (mittel-, weitporige) Kieselgele. Danach kann noch bis zu einem Feststoffgehalt von 95% zum Xerogel getrocknet. Kieselgele enthalten micellenartige Partikel von SiO₂ mit einem Durchmesser von 2 - 10 nm und spezifischen Oberflächen (BET) von 300 bis 1000 m²/g. Kieselgele werden in unterschiedlichen Qualitäten kommerziell angeboten, beispielsweise unter den Marken TriSyl^{®} oder Cab-O-Sil^{®}.

Das Kieselgel wird erfindungsgemäß vorzugsweise in Mengen von 0,001 bis 20 Gew.-%, insbesondere von 0,01 bis 2,0 Gew.-% und ganz besonders bevorzugt in Mengen von 0,1 bis 1,0 Gew.-%, jeweils bezogen auf die Fettstoffe als Bleichverstärker eingesetzt.

Die Temperatur, bei der der Bleichverstärker Verwendung findet ist vorzugsweise gegenüber der Raumtemperatur erhöht. Besonders bevorzugt kann es sein, die Reaktion mit dem Magnesiumsilikat bei Temperaturen von 60 bis 160°C und insbesondere von 80 bis 140 °C durchzuführen.

Fettstoffe im Sinne der vorliegenden technischen Lehre sind alle Verbindungen, die sich aus Fetten und Ölen ableiten lassen und die mindesten 6 C-Atome im Molekül aufweisen. Beispiele für Fettstoffe sind linearen, verzweigten, cyclischen, gesättigte oder ungesättigte Fettalkohole, Fettether, Fettsäure, Fettsäureester, vorzugsweise Fettsäureester des Glycerins, Alkyl(oligo)glycoside, Fettsäureamide, Fettsäureamine und/oder deren vorzugsweise alkoxylierte Derivate. Es sind aber auch andere Derivate umfasst, beispielsweise sulfatierte, sulfonierte oder hydroxylierte Derivate der oben beschriebenen Fettstoffe.

Bevorzugte Fettstoffe sind solche, die herstellungsbedingt einen unerwünscht hohen pH-Wert haben (z. B. bedingt durch alkalische Verunreinigungen). Z. B. sind bevorzugt solche Fettstoffe, die mindetstens eine Ethoxygruppe oder Propoxygruppe enthalten, oder die mindestens eine Oligoethoxygruppe oder Oligopropoxygruppe enthalten. Die Vorsilbe Oligo ist hier breit auszulegen. Oligo kann z. B. von 1 bis 1000, insbesondere von 2 bis 1000, bis 500, bis 100, bis 50 oder bis 10 Ethoxyeinheiten oder Propoxyeinheiten bezeichnen.

Besonders bevorzugt sind solche Fettstoffe, die oberflächenaktive Eigenschaften aufweisen, also als Tenside fungieren können. Vorzugsweise sind die Fettstoffe im Sinne der hier beschriebenen technischen Lehre anionische-, nichtionische-, kationische- oder amphotere Tenside. Diese sind in der folgenden Tabelle (nach **Römpp-Online, Version 2.10, 31.3.2006, Stichwort: "Tenside")** beispielhaft aufgeführt:

| **Klasse** | **typische Vertreter** |
|---|---|
| | |
| anion. Tenside. | Seifen |
| | Alkylbenzolsulfonate, Alkansulfonate |
| | Alkylsulfate |
| | Alkylethersulfate |
| nichtion. Tenside | Fettalkoholpolyglycolether, |
| | Alkylphenolpolyglycolether |
| | (ethoxylierte) Sorbitanfettsäureester, |
| | Alkylpolyglucoside, Fettsäureglucamide, |
| | Fettsäurepolyglycolester, Ethylenoxid-Propylenoxid- |
| | Blockpolymere, Polyglycerolfettsäureester, |
| | Fettsäurealkanolamide |
| kation. Tenside | quartäre Ammonium-Verb. mit einer od. zwei |
| | hydrophoben Gruppen, (z.B. |
| | Cetyltrimethylammoniumbromid u. |
| | Cetyltrimethylammoniumchlorid); |
| | Salze langkettiger prim. Amine |
| amphotere Tenside | *N*-(Acylamidoalkyl)betaine |
| | *N*-Alkyl- β-aminopropionate bzw. |
| | *N*-Alkyl-β-iminopropionate Amin-*N*-oxide |

Es kann weiterhin bevorzugt sein, solche Fettstoffe zu verwenden, die einen Schmelzpunkt von mehr als 21°C aufweisen, also bei Raumtemperatur fest sind. Da das hier beschriebene Verfahren vorzugsweise bei erhöhter Temperatur abläuft müssen geeignete Fettstoffe vorzugsweise im Bereich bis 120°C und insbesondere bis 140°C stabil bleiben ohne sich chemisch zu verändern. Bevorzugt sind daher Fettstoffe, die Schmelzpunkte von größer 21°C, vorzugsweise größer 80°C und besonders vorzugsweise größer 120°C und insbesondere größer/gleich 140°C aufweisen.

Die Bleiche selbst, also das Inkontaktbringen der Fettstoffe mit dem H₂O₂ erfolgt ebenfalls vorteilhafterweise und daher bevorzugt bei erhöhten Temperaturen. Besonders bevorzugt ist eine solche Verfahrensführung, bei der die Fettstoffe mit dem Bleichmittel bei Temperaturen von 60 bis 100 °C und vorzugsweise bei Temperaturen von 60 bis 90 °C behandelt werden. Besonders bevorzugte Substrate für das vorliegend beschriebene Bleichverfahren sind aber nichtionische Tenside und Fettstoffe, die im Folgenden im Einzelnen beschrieben werden:

### Hydroxymischether (HME):

Es handelt sich z.B. um handelsübliche Tenside der allgemeinen Formel (I)

R¹O[CH₂CH₂O]ₓCH₂CH(OM)R² (I)

in der R¹ für einen linearen oder verzweigten Alkyl- und/oder Alkenylrest mit 4 bis 22 Kohlenstoffatomen bedeutet, oder für einen Rest R²-CH(OH)CH₂ steht, wobei R² für einen linearen oder verzweigten Alkyl- und/oder Alkenylrest mit 8 bis 16 Kohlenstoffatomen, x für eine Zahl von 40 bis 80 steht, und M für ein Wasserstoffatom oder einen gesättigten Alkylrest mit 1 bis 18 Kohlenstoffatomen steht.

Vorteilhaft werden solche Verbindungen der allgemeinen Formel (I) verwendet, die mindestens eine freie Hydroxylgruppe (= -OH) enthalten. Bevorzugt im Sinne der Erfindung werden solche Hydroxymischether, die sich von Ethoxylaten von einwertigen Alkoholen der Formel R¹-OH mit 6 bis 18 Kohlenstoffatomen, vorzugsweise 6 bis 16 und insbesondere 8 bis 10 Kohlenstoffatomen ableiten, wobei R¹ für einen linearen Alkylrest steht und x für 40 bis 60. Weiterhin sind in den erfindungsgemäßen Mischungen solche Verbindungen der allgemeinen Formel (I) bevorzugt, bei denen der Index x für eine Zahl von 40 bis 70, vorzugsweise 40 bis 60 und insbesondere von 40 bis 50 steht. M ist dabei dann ein Wasserstoffatom. Ganz besonders bevorzugt sind Hydroxymischether der Formel (I), wobei R¹ für einen Alkylrest mit 8 bis 10 Kohlenstoffatomen, insbesondere auf Basis eines nativen Fettalkohols, R² für einen Alkylrest mit 10 Kohlenstoffatome, insbesondere für einen linearen Alkylrest und x für 40 bis 60 steht. Bevorzugt sind weiterhin Mischungen, die als oberflächenaktive Verbindung eine Verbindung nach der allgemeinen Formel (I) enthält, in der R¹ für eine Alkyl- und/oder Alkenylrest mit 8 bis 10 Kohlenstoffatomen steht und R² für einen Alkyl- oder Alkenylrest mit 8 bis 10 Kohlenstoffatomen steht und x eine Zahl von 40 bis 50 bedeutet, wobei auch hier M für ein Wasserstoffatom steht. Geeignet sind aber auch solche Verbindungen der Formel (I) bei denen R¹ für einen Alkyl- oder Alkenylrest mit 8 bis 10 Kohlenstoffatomen steht, R² für einen Rest mit 8 bis 12 Kohlenstoffatomen steht und M einen gesättigten Alkylrest mit 1 bis 6, vorzugsweise 1 bis 4 Kohlenstoffatomen bedeutet. Letztere Verbindung enthält keine freien Hydroxylgruppen - vielmehr wurden die HydroxylFunktionen mit geeigneten Reagenzien, z.B. Alkylhalogeniden alkyliert.

Des Weiteren sind HME-Verbindungen der Formel (II) geeignete Substrate:

R³O [CH₂CHCH₃O]_{z}[CH₂CH₂O]_{y}CH₂CH(OH)R⁴ (II)

in der R³ für einen linearen oder verzweigten Alkyl- und/oder Alkenylrest mit 8 bis 22 Kohlenstoffatomen, R⁴ für einen linearen oder verzweigten Alkyl- und/oder Alkenylrest mit 8 bis 16 Kohlenstoffatomen, y für eine Zahl von 10 und 35 steht, z Null bedeutet oder eine Zahl von 1 bis 5 sein muss. Es kann vorteilhaft sein dass, wenn R³ = R¹ und gleichzeitig R⁴ = R² solche Verbindungen der Formel (II) ausgewählt werden in denen der Index z mindestens 1 ist. Sofern Mischungen der oberflächenaktiven Verbindungen vom Typ der Formel (I) mit denen der Formel (II) Verwendung finden, sind nur solche Mischungen im Sinne der vorliegenden technischen Lehre, bei denen sich die Moleküle strukturell voneinander unterscheiden. Es müssen also immer strukturverschiedene Verbindungen nebeneinander vorliegen. Besonders bevorzugte Verbindungen sind beispielsweise solche bei denen in der Formel (II) der Index y für einen Zahl von 20 bis 30, vorzugsweise von 20. bis 25 steht. Weiterhin bevorzugt sind solche Verbindungen bei denen in der Formel (II) R³ einen Alkylrest mit 8 bis 12, vorzugsweise 8 bis 10 Kohlenstoffatomen repräsentiert, R⁴ für einen Alkylrest mit 10 bis 12, vorzugsweise mir 10 Kohlenstoffatomen bedeutet, y eine Zahl von 15 bis 35, vorzugsweise 20 bis 30 bedeutet und z eine Zahl von 1 bis 3, vorzugsweise 1 bedeutet. Bevorzugt sind auch Mischungen, die als oberflächenaktive Verbindungen solche nach der allgemeinen Formel (II) enthalten, in der R³ für eine Alkyl- und/oder Alkenylrest mit 11 bis 18 Kohlenstoffatomen steht und R⁴ für einen Alkyl- oder Alkenylrest mit 8 bis 10 Kohlenstoffatomen steht und y eine Zahl von 20 bis 35 bedeutet. Ebenfalls bevorzugt sind Mischungen, die als oberflächenaktive Verbindung nach der allgemeinen Formel (II) enthält, in der R³ für eine Alkyl- und/oder Alkenylrest mit 8 bis 12 Kohlenstoffatomen steht und R⁴ für einen Alkyl- oder Alkenylrest mit 8 bis 10 Kohlenstoffatomen steht und y eine Zahl von 20 bis 35 und z eine Zahl von 1 bis 3 bedeutet. Die Verbindungen des Typs (II) stellen ebenfalls Hydroxymischetherderivate dar, die durch Umsetzung von propoxylierten und/oder ethoxylierten Fettalkoholen mit Alkylepoxiden durch Ringöffnung im alkalischen Milieu hergestellt werden können. Dabei ist es bei Derivaten des Typs (II) wie auch bei allen anderen in dieser Beschreibung aufgeführten gemischten Alkoxylaten, die also sowohl einen Propylenoxid-Rest CH₂CHCH₃O (PO) als auch einen Ethylenoxid-Rest CH₂CH₂O (EO) enthalten, möglich, dass aus Richtung des C-Atoms mit der freien Hydroxylgruppe zunächst die EO-Reste und dann die PO-Reste blockweise angeordnet sind, wobei auch die Abfolge erst PO, dann EO möglich ist. Weiterhin können die Alkoxid-Gruppen auch statistisch verteilt (randomisiert) im Molekül vorliegen. Es ist auch möglich sowohl Block- als auch Random-Alkoxylate nebeneinander zu verwenden.

### Fettalkoholethoxylate

Bei Verbindungen handelt es sich um an sich bekannte Fettalkoholethoxylate der allgemeinen Formel (III) R⁵-(OC₂H₄)_{z}-OH, in der R⁵ für lineare oder verzweigte Alkyl- und/oder Alkenylreste mit 8 bis 22 Kohlenstoffatomen steht und z eine Zahl von 1 bis 20 und vorzugsweise von 1 bis 15, und insbesondere von 1 bis 10 steht. Typische Beispiele sind die Addukte von durchschnittlich 1 bis 20 Mol an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind Addukte von 10 bis 40 Mol Ethylenoxid an technische Fettalkohole mit 12 bis 18 Kohlenstoffatomen, wie beispielsweise Kökos-, Palm-, Palmkern- oder vorzugsweise Talgfettalkohol. Besonders bevorzugte Fettalkoholethoxylate basieren auf Talgalkohole, der mit 2 bis 10 und vorzugsweise 2 bis 5 Mol Ethylenoxid pro Mol Alkohol ethoxyliert sind.

### Glykolester

Diese. Verbindungen stellen Mono- und/oder vorzugsweise Diester des Glykols und insbesondere von Polyglykolen dar und sind ebenfalls bekannt und handelsüblich. Sie können mit der Formel R⁶CO-(OC₂H₄)ₘ-OR⁷ , beschrieben werden, wobei R⁶ für einen Alkyl- und/oder Alkenylreste mit 7 bis 21 Kohlenstoffatomen und m für Zahlen von 11 bis 100 steht, und R⁷ ein Wasserstoffatom oder einen Rest CO-R⁶ bedeutet. Dabei sind symmetrische (R⁶ = R⁷) und unsymmetrische Verbindungen (R⁶ ≠ R⁷) mit umfasst.

### Alkyl(oligo)glycoside

Diese Verbindungen sind als Alky(oligo)glycoside ebenfalls bekannt. Alkyl- und Alkenyloligoglykoside stellen bekannte nichtionische Tenside dar, die der Formel R⁸O-[G]ₚ folgen in der R⁸ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁸ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R⁸ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Betaine

Betaine stellen bekannte Tenside dar, die überwiegend durch Carboxyalkylierung, vorzugsweise Carboxymethylierung von aminischen Verbindungen hergestellt werden. Vorzugsweise werden die Ausgangsstoffe mit Halogencarbonsäuren oder deren Salzen, insbesondere mit Natriumchloracetat kondensiert, wobei pro Mol Betain ein Mol Salz gebildet wird. Ferner ist auch die Anlagerung von ungesättigten Carbonsäuren, wie beispielsweise Acrylsäure möglich. Beispiele für geeignete Betaine stellen die Carboxyalkylierungsprodukte von sekundären und insbesondere tertiären Aminen dar, die der folgenden Formel folgen: 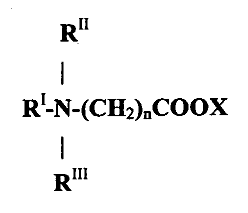 in der R¹ für Alkyl- und/oder Alkenylreste mit 6 bis 22 Kohlenstoffatomen, R^{II} für Wasserstoff oder Alkylreste mit 1 bis 4 Kohlenstoffatomen, R^{III} für Alkylreste mit 1 bis 4 Kohlenstoffatomen, n für Zahlen von 1 bis 6 und X für ein Alkali- und/oder Erdalkalimetall oder Ammonium steht. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosal-kyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische.

Weiterhin kommen auch Carboxyalkylierungsprodukte von Amidoaminen in Betracht, die der unten angegebenen Formel folgen, 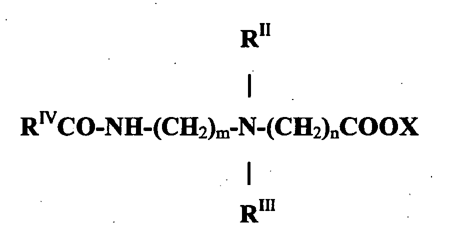 in der R^{IV}CO für einen aliphatischen Acylrest mit 6 bis 22 Kohlenstoffatomen und 0 oder 1 bis 3 Doppelbindungen, m für Zahlen von 1 bis 3 steht und R^{II}, R^{III}, n und X die oben angegebenen Bedeutungen haben. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von C_{8/18}-Kokosfettsäure-N,N-dimethylaminopropylamid mit Natriumchloracetat.

Weiterhin kommen als geeignete Ausgangsstoffe für die im Sinne der Erfindung einzusetzenden Betaine auch Imidazoline in Betracht, die dieser Formel folgen, 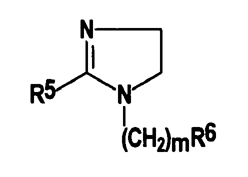 in der R^{V} für einen Alkylrest mit 5 bis 21 Kohlenstoffatomen, R⁶ für eine Hydroxylgruppe, einen OCOR^{V}- oder NHCOR^{V}-Rest und m für 2 oder 3 steht. Auch bei diesen Substanzen handelt es sich um bekannte Stoffe, die beispielsweise durch cyclisierende Kondensation von 1 oder 2 Mol Fettsäure mit mehrwertigen Aminen, wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der oben genannten Fettsäuren mit AEEA, vorzugsweise Imidazoline auf Basis von Laurinsäure oder wiederum C_{12/14}-Kokosfettsäure, die anschließend mit Natriumchloracetat betainisiert werden.

### Epoxide

Diese ebenfalls bekannten, nichtionischen Verbindungen werden beispielsweise hergestellt, indem man Alkyl-Epoxide mit Ethylenglykol und anschließend mit weiterem Ethylenoxid umsetzt. Es handelt sich ebenfalls um handelsübliche Substanzen. Sie folgen der allgemeinen Formel (IV) 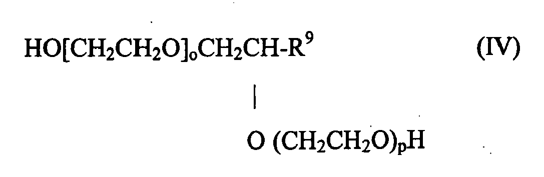 in der R⁹ für einen linearen oder verzweigten Alkyl- und/oder Alkenylrest mit 4 bis 22 Kohlenstoffatomen und o für eine Zahl von 1 bis 20 und der Index p für Null oder Zahlen von 1 bis 20 steht.
Diese ebenfalls als Hydroxymischether zu bezeichnenden Verbindungen folgen der allgemeinen Formel (V):

R¹⁰CH(OR¹¹)CH₂-OR¹¹ (V)

in der R¹⁰ für einen gesättigten oder ungesättigten, verzweigten oder unverzweigten Alkyl- oder Alkenylrest mit 8 bis 16 Kohlenstoffatomen steht, und R¹¹ jeweils unabhängig voneinander einen Rest (CH₂CH₂O)ᵣCH₂CH(OH)R¹² symbolisieren, wobei r in jedem Rest R¹¹ unabhängig für Null oder eine Zahl von 1 bis 50 steht und R¹² einen gesättigten oder ungesättigten, verzweigten oder unverzweigten Alkyl- oder Alkenylrest mit 8 bis 16 Kohlenstoffatomen steht.

### Amine

Es handelt sich dabei um stickstoffhaltige Verbindungen der allgemeinen Formel (VI):

NR¹³₃ (VI)

wobei R¹³ unabhängig voneinander für einen Rest (CH₂CH₂O)ₛ-CH₂CH(OH)R¹⁴ oder einen Alkylrest mit 8 bis 16 Kohlenstoffatomen steht und s für jeden einzelnen Rest R¹³ unabhängig Null bedeutet, oder eine Zahl von 1 bis 50. Verbindungen des Typs b8) sind beispielsweise erhältlich durch Ethoxylierung von Alkylaminen oder von Triethanolamin und anschließender Umsetzung mit Alkylenoxiden mit Alkylketten mit 8 bis 18 Kohlenstoffatomen unter Bedingungen der alkalischen Katalyse.

### Fettalkohole

Neben den oben beschrieben oberflächenaktiven Verbindungen kann es vorteilhaft sein, weitere oberflächenaktive Verbindungen als Substrate zu verwenden. Hier kommen insbesondere reine Fettalkohole in Frage. Unter Fettalkoholen sind primäre aliphatische Alkohole der Formel R¹⁴-OH zu verstehen, in der R für einen aliphatischen, linearen oder verzweigten Kohlenwasserstoffrest mit 6 bis 22 Kohlenstoffatomen und 0 und/oder 1, 2 oder 3 Doppelbindungen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol.

### Fettsäuren

Unter Fettsäuren sind aliphatische Carbonsäuren der Formel (VII) zu verstehen,

R¹⁵ CO-OH (VII)

in der R¹⁵CO für einen aliphatischen, linearen oder verzweigten Acylrest mit 6 bis 22 Kohlenstoffatomen und 0 und/oder 1, 2 oder 3 Doppelbindungen steht. Typische Beispiele sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen, wie beispielsweise Kokos-, Palm-, Palinkern- oder Talgfettsäure.

Die Hydroxymischether der obigen Beschreibung der Formel (I) und (II) sind besonders bevorzugte Substrate im Sinne der vorliegenden technischen Lehre.

### Beispiele

Zur Prüfung des erfindungsgemäßen Verfahrens wurden Bleichversuche mit und ohne Kieselgel an verschiedenen nicht-ionischen Tensiden des Typs HME (siehe Beschreibung oben) als Beispiele für Fettstoffe durchgeführt. Es wurde die folgenden Tenside (alle Fa. Cognis) untersucht:
- Tensid 1: modifizierter Fettalkoholpolyglykolether (Dehypon E127 - Fa. Cognis)
- Tensid 2: Hydroxymischether (Dehypon KE 3697 - Fa. Cognis)
- Tensid 3: modifizierter Fettalkoholpolyglykolether (Dehypon KE 3447 - Fa. Cognis)

### Beispiel 1

198,8 g eines modifizierten Fettalkoholpolyglykolethers (Tensid 1, 2 oder 3) wurden unter Rühren auf 60°C aufgeheizt. Dann wurde 1 g amorphes Kieselgel (TriSyl^{®}) zugegeben und die Mischung danach noch eine Stunde lang gerührt. Anschließend wurde die Mischung über einen beheizbaren Filter (T120, Fa. Seitz) bei 60 °C filtriert. Das Filtrat (168,4 g) wurden bei 60°C anschließend mit 0,48 g einer 35 %igen wässerigen H₂O₂-Lösung versetzt und 5 Stunden lang gerührt.
Die Beispiele 2 und 3 wurden anlog zu dem Beispiel 1 durchgeführt. Es wurden die Farbzahlen der Ausgangs- wie der Endprodukte mit einem Farbzahlbestimmungsgerät der Fa. Dr. Lange, Modell Lico 200 bestimmt. Zum Vergleich wurden auch Werte gemessen für eine Bleiche mit H₂O₂ aber ohne Zusatz von Kieselgel. Die Ergebnisse sind in der folgenden Tabelle 1 aufgeführt.

**Tabelle 1**

| Versuch / Tensid | **1** | **2** | **3** |
|---|---|---|---|
| Farbzahl nach Hazen | | | |
| vor Bleichung | 478 | 567 | >1000 |
| nach Behandlung mit Kieselgel | 48 | 33 | 87 |
| Bleiche ohne Zusatz von Kieselgel | 472 | 213 | 143 |

Man erkennt die deutliche Verbesserung der Farbe der Produkte nach der Bleiche. Deutlich wird aber auch, dass der Zusatz von amorphem Kieselgel zu einer weiteren, signifikanten Verbesserung der Farbzahl führt. Außerdem kann der bleichverstärkende Effekt bei niedrigeren Temperaturen erzielt werden, verglichen mit anderen Bleichverstärkern.

### pH-Wert-Absenkung:

Die pH-Werte während des Versuchs 3 wurden bestimmt. Es wurden die folgenden pH-Werte vor und nach Behandlung mit Kieselgel gemessen:

| Versuch 3 | Farbzahl nach Hazen | pH |
|---|---|---|
| vor Bleichung | >1000 | 10,5 |
| nach Behandlung mit Kieselgel | 87 | 7,5 |

Die in dem Beispiel behandelten Tenside waren keine Verkaufsprodukte, sondern Roh-Lösungen, erhalten aus der Reaktion zur Herstellung der Tenside. Bevor diese zum Verkauf geeignet sind müssen sie gebleicht und muss der pH-Wert abgesenkt werden.

## Patentansprüche

1. Ein Verfahren zur Behandlung von Fettstoffen, die mindestens 6 C-Atome im Molekül aufweisen, **dadurch gekennzeichnet, dass** man die Fettstoffe mit einem H₂O₂ enthaltenden Bleichmittel sowie mit Kieselgel in Kontakt bringt.

2. Das Verfahren nach Anspruch 1, **dadurch gekennzeichnet, dass** die Fettstoffe ausgewählt sind aus linearen, verzweigten, cyclischen, gesättigten oder ungesättigten Fettalkoholen, Fettethern, Fettsäuren, Fettsäureestern, vorzugsweise Fettsäureester des Glycerins, Alkyl(oligo)glycosiden, Fettsäureamiden, Fettsäureaminen und deren alkoxylierten Derivaten.

3. Das Verfahren nach einem der Ansprüche 1 oder 2 **dadurch gekennzeichnet, dass** die Fettstoffe oberflächenaktive Verbindungen sind.

4. Das Verfahren nach einem der Ansprüche 1 bis 3, **dadurch gekennzeichnet, dass** die Fettstoffe nichtionische, anionische, kationische und/oder amphotere Tenside darstellen.

5. Das Verfahren nach einem der Ansprüche 1 bis 4, **dadurch gekennzeichnet, dass** die Fettstoffe ausgewählt sind aus der Gruppe der Hydroxymischether.

6. Das Verfahren nach einem der Ansprüche 1 bis 5, **dadurch gekennzeichnet, dass** in einem ersten Schritt die erwärmte oberflächenaktive Verbindung mit Kieselgel behandelt und anschließend mit dem H₂O₂ enthalten Bleichmittel gebleicht wird.

7. Das Verfahren nach einem der Ansprüche 1 bis 6 **dadurch gekennzeichnet, dass** die Fettstoffe gleichzeitig mit Kieselgel und dem H₂O₂ enthalten Bleichmittel behandelt wird.

8. Das Verfahren nach einem der Ansprüche 1 bis 7, **dadurch gekennzeichnet, dass** als H₂O₂ enthaltendes Bleichmittel eine wässerige alkalische Lösung von H₂O₂ verwendet wird.

9. Das Verfahren nach einem der Ansprüche 1 bis 8, **dadurch gekennzeichnet, dass** das Kieselgel in Mengen von 0,001 bis 20 Gew.-%, vorzugsweise von 0,01 bis 2,0 und insbesondere von 0,1 bis 1,0 Gew.-% - bezogen auf die Menge an Fettstoffen - eingesetzt werden.

10. Das Verfahren nach einem der Ansprüche 1 bis 9, **dadurch gekennzeichnet, dass** die Fettstoffe bei Temperaturen von 50 bis 140°C, vorzugsweise bei Temperaturen von 60 bis 100°C gebleicht und mit Kieselgel behandelt werden.

11. Das Verfahren nach einem der Ansprüche 1 bis 10, **dadurch gekennzeichnet, dass** die Behandlung der Fettstoffe mit dem H₂O₂ enthaltenden Bleichmittel bei Temperaturen von 60 bis 100 °C und vorzugsweise bei 60 bis 90 °C erfolgt.

12. Die Verwendung einer Kombination aus einem H₂O₂ enthaltenden Bleichmittel und Kieselgel zur Erniedrigung eines unerwünscht hohen pH-Wertes und zur Bleichung von Fettstoffen.

13. Die Verwendung nach Anspruch 12 in dem Verfahren nach einem der Ansprüche 1 bis 11.
